# EUROPEAN PATENT SPECIFICATION

(11) **EP 3 104 833 B1**
(45) Date of publication and mention of the grant of the patent: **03.01.2018**
(21) Application number: 15702469.6
(22) Date of filing: 03.02.2015
(51) Int. Cl.: A61K 47/18, A61K 8/44, A61Q 19/00

(54) **CAPRYLOYL ALANINE ETHYL ESTER AS A PENETRATION ENHANCEER**
CAPRYLOYL-ALANIN-ETHYLESTER ALS EIN PENETRATIONSFÖRDERER
ÉTHYL ESTER DE CAPRYLOYL ALANINE COMME AMPLIFICATEUR DE PÉNÉTRATION

(30) Priority: 13.02.2014 EP 14154961
(43) Date of publication of application: 21.12.2016
(73) Proprietor: BASF SE, 67056 Ludwigshafen am Rhein (DE); BASF France SAS, 92300 Levallois-Perret (FR)
(72) Inventor: MATHIS, Raymond, 40627 Düsseldorf (DE); RATHS, Hans-Christian, 40789 Monheim (DE); MEHLING, Annette, 42349 Wuppertal (DE); BEUCHÉ, Marc, F-91430 Vauhallan (FR)
(74) Representative: BASF IP Association
(86) International application number: PCT/EP2015/052109
(87) International publication number: WO 2015/121102

(56) References cited:
- EP-A1- 0 552 405
- EP-A1- 1 269 980
- EP-A1- 1 277 462
- EP-A2- 0 928 608
- WO-A1-01/35998

## Description

### 1. Field of Invention

The present invention relates to the use of caprylol-alanine ethylester as penetration enhancer in cosmetic or other topical compositions and cosmetic compositions comprising caprylol-alanine ethylester in combination with active ingredients, in particular those having skin benefits such as UV-filters, anti-acne actives, anti-aging actives, anti-inflammatory agents, hair modulating agents, antiperspirant or deodorizing agents, whitening agents, self-tanning agents and vitamins.

### 2. Description of the Prior Art

Pharmaceutical transdermal therapeutic systems were the first area of application for percutaneous absorption promoters also called penetration enhancers before their use in cosmetic compositions started. The delivery of pharmaceutical actives through the skin is of high interest because it enables systemic drug delivery avoiding the hepatic first pass mechanism. However, the barrier properties of the skin impede the passage of most molecules through the skin. Penetration enhancers can be used to promote the absorption of the substance of interest. Different chemical groups have been investigated for their penetration enhancing activity and a number of modes of action and sites of interaction have been identified. Among the mechanisms of action are penetration enhancing effect by 1) adapting the solvent properties to the substance of interest to improve partitioning into the skin, 2) interacting with the intercellular lipid matrix thereby altering the barrier structure, 3) modifying the intercellular proteins to increase permeability and/or 4) creating reservoir effects within the skin.

One of the first skin penetration enhancers described and used in pharmaceutical compositions was dimethylsulphoxide (DMSO), a universal solvent belonging to the sulphoxides.

The first substance specifically designed to be a skin penetration enhancer was the azone laurocapran, a very lipophilic substance which improves penetration by interacting with the lipids in the stratum corneum.

Pyrrolidones such as N-methyl-2-pyrrolidone (NMP) have also been used for the transdermal absorption enhancement of anti-inflammatory drugs, but have toxicological concerns which preclude their use.

Use of terpenes such as menthol has also been described whereby odor may make this use challenging. Menthol increases the solubility of the penetrating drugs by creating an eutectic system with these actives but also increases penetration by changing the barrier properties of the stratum corneum by its interaction with the lipid domains. Fatty acids, mainly unsaturated fatty acids (oleic acid), have successfully been used as enhancers. Since they are very lipophilic they modify the lipid domains of the stratum corneum, while alcohols such as ethanol or lauryl alcohol dissolve and extract the lipids of the stratum corneum and interact with the lipid bilayer, so that they improve penetration mainly of hydrophilic molecules.

In recent years more and more active ingredients are being used in cosmetics. Due to the topical application route, penetration enhancers have also found their way into personal care applications. The main focus of applying penetration enhancers in the cosmetic field is not the systemic delivery of a substance, but to increase the penetration of the active into the skin to enhance the efficacy of the cosmetic. Typically penetration enhancers such as solvent systems e.g. propylene glycol, which act by increasing drug partitioning or agent facilitating hydration of the stratum corneum, are used in cosmetic compositions.

As a rule cosmetic products need to have good skin compatibility and the ingredients should have a good toxicological profile.

Various investigations have shown that amino acid esters are effective penetration enhancers with a low toxicity and very good biocompatibility.

The European patent application EP 0 552 505 A1 discloses a percutaneous absorption enhancer for pharmacologically active substances comprising a derivative of an amino acid having the formula: R¹NH-R³ -COOR² wherein R¹ is a hydrogen atom, an acyl group having 1 to 20 carbon atoms or a hydrocarbon group having 1 to 20 carbon atoms, R² is a hydrogen atom or a hydrocarbon group having 1 to 20 carbon atoms and R³ is -CHR⁴- or benzyl, R⁴ being a hydrogen atom, methyl group, isopropyl group, 2-methylpropyl group or 1-methylpropyl group. The most effective candidates of the suggested absorption enhancers for pharmacologically active substances selected from the group consisting of indomethacin, ketoprofen, prednisolone and pindolol were esters of N-n-dodecanoylglycine. Ethylesters of dodecanoyl-aminoacids such as alanine, valine, leucin and isoleucine have as well been tested.

N-lauroyl-isopropylsarcosinate was subject of various patent applications in the cosmetic field:
It is the candidate of choice as outlined in the International patent family of WO 2003/039448 to dissolve many components and actives of the lipid phase in cosmetic and dermatological compositions thickened with a polymer. The described polymers have an average molecular weight below 100000 DA and preferably ranging between 1000 and 30000, comprising: a) a polymeric backbone, having repeating hydrocarbon units provided with at least a heteroatom, and b) optionally at least a pendant fatty chain and/or at least a terminal fatty chain, optionally functionalized, having 12 to 120 carbon atoms, bound to said units, the fatty chains representing 40 to 98 percent of the total number of the heteroatom units and of the fatty chains. These compositions could also contain UV-filters.

Subject of the European application EP 1277462 A1 were cosmetic or dermatological compositions intended for artificially tanning skin, comprising at least one self-tanning agent and at least one N-acyl amino acid ester. It had been shown that the addition of an N-acyl amino acid ester was improving the coloration and stability of a self-tanning agent in a composition for artificially tanning the skin. Isopropyl N-lauroyl sarcosinate was chosen in combination with self-tanning agents.

A composition comprising at least one 1,3,5-triazine derivative and at least one N-acyl amino acid ester and its use in or for the manufacture of cosmetic compositions for protecting the skin, the lips or integuments against ultraviolet radiation was disclosed in the European application EP 1 269 980 A1**.** The chosen N-acyl amino acid ester in combination with a 1,3,5-triazine derivative was isopropyl N-lauroylsarcosinate which was improving the sun protection factor of the composition.

The invention of US application US 2005/0065251 A1 concerns a cosmetic composition structured by a polyamide, characterized in that it comprises at least an organic UV filter, and at least an amino acid N-acylated ester, its use in or for preparing compositions for protecting the skin, the lips or skin appendages against ultraviolet radiation. The preferred amino acid N-acetylated ester was once again isopropyl N-lauroylsarcosinate.

Use of isopropyl N-lauroylsarcosinate has a number of limitations. It is a semisolid compound with a waxy like texture which is difficult to incorporate into the skin composition as its melting point is above room temperature. This melting point also precludes the substance from melting on the skin.

Objective of the present invention is providing an effective penetration enhancer for topical compositions with a good tolerability and improved compatibility with other cosmetic additives, which can easily be incorporated into cosmetic compositions. The penetration enhancer also can provide a good sensory profile.

### 3. Description of the invention

The present invention is related to the use of caprylol-alanine ethylester as penetration enhancer in cosmetic and other topical or oral compositions. In the context of this invention, penetration can also include permeation and absorption.

One embodiment of the current invention is directed to the cosmetic composition comprising capryloyl alanine ethylester as a penetration enhancer. These compositions contain the caprylol-alanine ester in combination with an active, preferably in combination with UV-filters, anti-acne actives, anti-aging actives, anti-inflammatory agents, hair modulating agents, antiperspirant or deodorizing agents, whitening agents, self-tanning agents and vitamins. Surprisingly, compositions comprising caprylol-alanine ethylester were found to provide a very good sensory profile whilst imparting a moisturizing feeling to the skin without unpleasant tackiness. Caprylol-alanine ethylester also promotes the skin penetration of actives more than other N-acyl-amino acid esters, especially the penetration of hydrophilic actives.

Cosmetic compositions could contain 0.1 to 90 weight % of capryloyl alanine ethylester based on the total composition, typically would contain 1 to 30 weight %, preferably 2 to 10 weight % and more preferably 2 to 5 weight % based on the total composition.

Capryloyl alanine ethyl ester turned out to have a smaller Hansen Solubility Parameters (HSP) distance to the stratum corneum than well-known and established references like DMI and ethyldiethylenglycol which indicates a good potential as skin penetration enhancer. Hansen Solubility Parameters are a key indicator for good partitioning into the stratum corneum provided the molecule is liquid at RT and has a small enough molecular volume. The addition of caprylol-alanine ethylester as penetration enhancer has many advantages for cosmetic compositions containing actives. The skin compatibility of formulations containing actives with disadvantageous compatibility properties could be improved especially as the targeted effect is either achieved ore quickly and/or the desired tissue (layer) is more efficiently targeted and/or a lower concentration of active ingredient can be used.

The penetration enhancer itself penetrates through the skin to a lesser degree than DMI and therefore is less likely to be systemically available. Furthermore, capryloyl-alanine ethylester can be metabolized in the skin thereby contributing to an improved toxicological profile.

In addition capryloyl-alanine ethylester is perceived to have a moisturizing effect similar to other emollients, is perceived to be absorbed more quickly than other emollients so that its application in a topical composition results in a pleasant sensory profile.

Caprylol-alanine ethylester is an odorless liquid substance and as such very easily incorporated into cosmetic and topical compositions. The resulting formulations have a very good physico-chemical stability. As a polar emollient, it has beneficial solubilizing properties and can improve the dispersion and distribution of actives on tissues.

### Active compounds

The formulations according to the invention comprise caprylol-alanine ester in combination with cosmetically acceptable active compounds.

According to the invention, the active compounds (one or more compounds) can advantageously be chosen from the group consisting of UV-filters, anti-acne actives, anti-aging actives, anti-inflammatory agents, hair growth modulating agents, agents with effects on the hair or hair follicle, antiperspirant or deodorizing agents, whitening agents, self-tanning agents, peptides, oligopeptides, proteins, acetylsalicylic acid, atropine, azulene, hydrocortisone and derivatives thereof, e.g. hydrocortisone 17-valerate, vitamins, especially of the B and D series, in particular vitamin B1, vitamin B12, vitamin D, vitamin A and derivatives thereof, such as retinyl palmitate, vitamin E or derivatives thereof, such as e.g. tocopheryl acetate, vitamin C and derivatives thereof, such as e.g. ascorbyl glucoside, and also niacinamide, panthenol, bisabolol polydocanol, unsaturated fatty acids, such as e.g. the essential fatty acids (conventionally called vitamin F), in particular γ-linolenic acid, oleic acid, eicosapentaenoic acid, docosahexaenoic acid and derivatives thereof, chloramphenicol, caffeine, prostaglandins, thymol, camphor, squalene, extracts or other products of plant and animal origin, e.g. evening primrose oil, borage oil or carob bean oil, fish oils, cod-liver oil, and also ceramides and ceramide-like compounds, incense extract, green tea extract, water lily extract, liquorice extract and witch hazel, antidandruff active compounds (e.g. selenium disulfide, zinc pyrithione, piroctone, olamine, climbazole, octopirox, polydocanol and combinations thereof), and complexed active compounds, such as e.g. those of γ-oryzanol and calcium salts, such as calcium pantothenate, calcium chloride and calcium acetate.

The active compound or compounds can also be chosen from the group consisting of NO synthase inhibitors, in particular if the compositions according to the invention are to be used for treatment and prophylaxis of the symptoms of intrinsic and/or extrinsic ageing of the skin and for treatment and prophylaxis of the harmful effects of ultraviolet radiation on the skin and hair. Nitroarginine is the preferred NO synthase inhibitor.

The active compound or compounds are furthermore advantageously chosen from the group including catechols and bile acid esters of catechols and aqueous or organic extracts from plants or plant parts which have a content of catechols or bile acid esters of catechols, such as, for example, the leaves of the Theaceae plant family, in particular of the species Camellia sinensis (green tea). Catechols are a group of compounds which are to be interpreted as hydrogenated flavones or anthocyanidines and are derivatives of "catechol" (catechol, 3,3',4',5,7-flavanepentaol, 2-(3,4-dihydroxyphenyl)-chromane-3,5,7-triol). Epicatechol ((2R,3R)-3,3',4',5,7-flavanepentaol) is also an advantageous active compound in the context of the present invention.

Preferred active compounds are furthermore polyphenols or catechols from the group consisting of (-)-catechol, (+)-catechol, (-)-catechol gallate, (-)-gallocatechol gallate, (+)-epicatechol, (-)-epicatechol, (-)-epicatechol gallate, (-)-epigallocatechol and (-)-epigallocatechol gallate.

Flavone and its derivatives (often also collectively called "flavones") are also advantageous active compounds in the context of the present invention. Further advantageous active compounds are sericoside, pyridoxol, vitamin K, biotin and aroma substances.

Preferably the active compounds (one or more compounds) can very advantageously be chosen from the group consisting of hydrophilic active compounds α-hydroxy acids, such as lactic acid or salicylic acid, or salts thereof, such as e.g. Na lactate, Ca lactate or TEA lactate, urea, allantoin, serine, sorbitol, glycerol, milk proteins, panthenol or chitosan.

Most preferably the active compounds are chosen from the group selected from UV-filters, anti-acne actives, anti-aging actives, anti-inflammatory agents, hair growth modulating agents, agents with effects on the hair or hair follicle, antiperspirant or deodorizing agents, whitening agents, self-tanning agents and vitamins.

### a) UV-filters

It was investigated that the chosen penetration enhancer remarkably improves solubilization of UV-filters and thus greatly increases the UV-protecting properties of the inventive cosmetic compositions.

Caprylol-alanine ethylester has interesting UV filter solubilizing properties. Known UV-filters such as ethylhexyltriazone (Uvinul T150) can be better solubilized than in other typically used solvents, for example 23 wt % compared to 16 wt % in dibutyl adipate, thereby increasing the concentrations achievable in the solvent . This in turn can be beneficial for designing formulations with improved sensorial profiles. A more homogenous distribution and better penetration into the upper layers of the stratum corneum can also be achieved.

In addition, the UV-protecting effect of UV-filters which are pigments and act physically is also improved by adding caprylol-alanine ethylester. Due to the enhancer's excellent spreading properties these kind of UV-filters have an improved homogenous distribution in the cosmetic composition and on the skin after application.

Hence the active compounds (one or more compounds) can furthermore very advantageously be chosen from the group consisting of light filter active compounds.

Suitable light filter active compounds are substances which absorb UV rays in the UV-B and/or UV-A range. By these there are to be understood organic substances which are capable of absorbing ultraviolet rays and of releasing the energy absorbed again in the form of longer-wavelength radiation, e.g. heat. The organic substances can be oil-soluble or water-soluble. Suitable UV filters are e.g. 2,4,6-triaryl-1,3,5-triazines, in which the aryl groups in each case can carry at least one substituent, which is preferably chosen from hydroxyl, alkoxy, specifically methoxy, and alkoxycarbonyl, specifically methoxycarbonyl and ethoxycarbonyl. p-Aminobenzoic acid esters, cinnamic acid esters, benzophenones, camphor derivatives and pigments which keep out UV rays, such as titanium dioxide, talc and zinc oxide, are furthermore suitable. Pigments based on titanium dioxide are particularly preferred.

Oil-soluble UV-B filters which can be used are e.g. the following substances: 3-benzylidenecamphor and derivatives thereof, e.g. 3-(4-methylbenzylidene)camphor; 4-aminobenzoic acid derivatives, preferably 4-(dimethylamino)-benzoic acid 2-ethyl¬hexyl ¬ester, 4-(dimethylamino)benzoic acid 2-octyl ester and 4-(dimethylamino)benzoic acid amyl ester; esters of cinnamic acid, preferably 4-methoxycinnamic acid 2-ethylhexyl ester, 4-methoxycinnamic acid propyl ester, 4-methoxycinnamic acid isoamyl ester, 4 methoxycinnamic acid isopentyl ester and 2-cyano-3-phenyl-cinnamic acid 2-ethylhexyl ester (octocrylene); esters of salicylic acid, preferably salicylic acid 2-ethylhexyl ester, salicylic acid 4-isopropylbenzyl ester and salicylic acid homomenthyl ester; derivatives of benzophenone, preferably 2-hydroxy-4-methoxybenzophenone, 2-hydroxy-4-methoxy-4'-methylbenzophenone and 2,2'-dihydroxy-4-methoxybenzophe¬none; esters of benzalmalonic acid, preferably 4-methoxybenzalmalonic acid di-2-ethylhexyl ester; triazine derivatives, such as e.g. 2,4,6-trianilino-(p-carbo-2'-ethyl-1'-hexyloxy)-1,3,5-triazine (octyl¬triazone) and dioctyl butamido triazone ; propane-1,3-diones, such as e.g. 1-(4-tert-butylphenyl)-3-(4'-methoxyphenyl)propane-1,3-dione.

Possible water-soluble substances are:
- 2-phenylbenzimidazole-5-sulfonic acid and alkali metal, alkaline earth metal, ammonium, alkylammonium, alkanolammonium and glucammonium salts thereof;
- sulfonic acid derivatives of benzophenones, preferably 2-hydroxy-4-methoxybenzophenone-5-sulfonic acid and its salts;
- sulphonic acid derivatives of 3-benzylidenecamphor, such as e.g. 4-(2-oxo-3-bornylidenemethyl) benzenesulphonic acid and 2-methyl-5-(2-oxo-3-bornylidene)sulphonic acid and salts thereof.

The use of esters of cinnamic acid, preferably 4-methoxycinnamic acid 2-ethylhexyl ester, 4-methoxycinnamic acid isopentyl ester and 2-cyano-3-phenyl-cinnamic acid 2-ethylhexyl ester (octocrylene), is particularly preferred.

The use of derivatives of benzophenone, in particular 2-hydroxy-4-methoxybenzophenone, 2-hydroxy-4-methoxy-4'-methylbenzophenone and 2,2'-dihydroxy-4-methoxybenzophe¬none, and the use of propane-1,3-diones, such as e.g. 1-(4-tert-butylphenyl)-3-(4'-methoxyphenyl)propane-1,3-dione, is furthermore preferred.

Possible typical UV-A filters are:
- derivatives of benzoylmethane, such as, for example, 1-(4'-tert-butylphenyl)-3-(4'-methoxyphenyl)-propane-1,3-dione, 4-tert-butyl-4'-methoxydibenzoylmethane or 1-phenyl-3-(4'-isopropylphenyl)-propane-1,3-dione;
- amino-hydroxy-substituted derivatives of benzophenones, such as e.g. N,N-diethylamino-hydroxybenzoyl-n-hexyl-benzoate.

The UV-A and UV-B filters can of course also be employed in mixtures.

Further suitable UV filter substances are mentioned in the following table:

| No. | Substance | CAS no. (=acid) |
|---|---|---|
| 1 | 4-aminobenzoic acid | 150-13-0 |
| 2 | 3-(4'-trimethylammonium)-benzylidenebornan-2-one methylsulfate | 52793-97-2 |
| 3 | 3,3,5-trimethyl-cyclohexyl salicylate (homosalatum) | 118-56-9 |
| 4 | 2-hydroxy-4-methoxy-benzophenone (oxybenzonum) | 131-57-7 |
| 5 | 2-phenylbenzimidazole-5-sulfonic acid and its potassium, sodium and triethanolamine salts | 27503-81-7 |
| 6 | 3,3'-(1,4-phenylenedimethine)-bis(7,7-dimethyl-2-oxobicyclo[2.2.1]heptane-1-methanesulfonic acid) and its salts | 90457-82-2 |
| 7 | 4-bis(polyethoxy)amino-benzoic acid polyethoxy-ethyl ester | 113010-52-9 |
| 8 | 4-dimethylamino-benzoic acid 2-ethylhexyl ester | 21245-02-3 |
| 9 | salicylic acid 2-ethylhexyl ester | 118-60-5 |
| 10 | 4-methoxy-cinnamic acid 2-isoamyl ester | 71617-10-2 |
| 11 | 4-methoxy-cinnamic acid 2-ethylhexyl ester | 5466-77-3 |
| 12 | 2-hydroxy-4-methoxy-benzophenone-5-sulfonic acid (sulisobenzonum) and the sodium salt | 4065-45-6 |
| 13 | 3-(4'-sulfobenzylidene)-bornan-2-one and salts | 58030-58-6 |
| 14 | 3-benzylidenebornan-2-one | 16087-24-8 |
| 15 | 1-(4'-isopropylphenyl)-3-phenylpropane-1,3-dione | 63260-25-9 |
| 16 | 4-isopropylbenzyl salicylate | 94134-93-7 |
| 17 | 3-imidazol-4-yl-acrylic acid and its ethyl ester | 104-98-3 |
| 18 | 2-cyano-3,3-diphenylacrylic acid ethyl ester | 5232-99-5 |
| 19 | 2-cyano-3,3-diphenylacrylic acid 2'-ethylhexyl ester | 6197-30-4 |
| 20 | menthyl-o-aminobenzoate or: 5-methyl-2-(1-methylethyl)-2-aminobenzoate | 134-09-8 |
| 21 | glyceryl p-aminobenzoate or: 4-aminobenzoic acid 1-glyceryl ester | 136-44-7 |
| 22 | 2,2'-dihydroxy-4-methoxybenzophenone (dioxybenzone) | 131-53-3 |
| 23 | 2-hydroxy-4-methoxy-4-methylbenzophenone (mexenone) | 1641-17-4 |
| 24 | triethanolamine salicylate | 2174-16-5 |
| 25 | dimethoxyphenylglyoxalic acid or: sodium 3,4-dimethoxy-phenyl-glyoxalate | 4732-70-1 |
| 26 | 3-(4'-sulfobenzylidene)-bornan-2-one and its salts | 56039-58-8 |
| 27 | 4-tert-butyl-4'-methoxy-dibenzoylmethane | 70356-09-1 |
| 28 | 2,2',4,4'-tetrahydroxybenzophenone | 131-55-5 |
| 29 | 2,2'-methylene-bis-[6-(2H-benzotriazol-2-yl)-4-(1,1,3,3,-tetramethylbutyl)phenol] | 103597-45-1 |
| 30 | 2,2'-(1,4-phenylene)-bis-1H-benzimidazole-4,6-disulfonic acid, Na salt | 180898-37-7 |
| 31 | 2,4-bis-[4-(2-ethylhexyloxy)-2-hydroxy]phenyl-6-(4-methoxyphenyl)-(1,3,5)-triazine | 187393-00-6 |
| 32 | 3-(4-methylbenzylidene)-camphor | 36861-47-9 |
| 33 | 4-bis(polyethoxy)paraaminobenzoic acid polyethoxyethyl ester | 113010-52-9 |
| 34 | 2,4-dihydroxybenzophenone | 131-56-6 |
| 35 | 2,2'-dihydroxy-4,4'-dimethoxybenzophenone-5,5'-disodium-sulfonate | 3121-60-6 |
| 36 | 2-[4-(diethylamino)-2-hydroxybenzoyl]-benzoic acid hexyl ester | 302776-68-7 |
| 37 | 2-(2H-benzotriazol-2-yl)-4-methyl-6-[2-methyl-3-[1,3,3,3-tetramethyl-1-[(trimethylsilyl)oxy]disiloxanyl]propyl]phenol | 155633-54-8 |
| 38 | 1,1-[(2,2'-dimethylpropoxy)carbonyl]-4,4-diphenyl-1,3-butadiene | 363602-15-7 |

In addition to the two abovementioned groups of primary sunscreen substances, secondary sunscreen agents of the antioxidant type, which interrupt the photochemical and cellular reaction chain triggered when UV radiation penetrates into the skin, can also be employed. Typical examples are the UV induced functional and/or structural changes in superoxide dismutase, catalase, tocopherols (vitamin E) and ascorbic acid (vitamin C).

A further group of suitable actives are anti-inflammatory substances, which have a beneficial action on skin damaged by UV light. Such substances are, for example, but not limited to, bisabolol, phytol and phytantriol and acetyl salicylic acid.

The list of active compounds and active compound combinations mentioned which can be used in the compositions according to the invention is of course not intended to be limiting. The active compounds can be used individually or in any desired combinations with one another.

The amount of such active compounds (one or more compounds) in the compositions according to the invention is preferably 0.001 to 30 wt.%, particularly preferably 0.05 to 20 wt.%, in particular 1 to 10 wt.%, based on the total weight of the composition.

### b) Self-tanning agents and whitening agents

In addition caprylol-alanine ethylester improves the evenness of self-tanning agents. Due to the addition of the penetration enhancer the self-tanning agents and whitening agents can be applied much more evenly and result in a homogenous skin tanning or skin whitening effect. Suitable self-tanning agents are pyrazoline-4,5-dione derivatives, glutaraldehyde, alloxan, ninhydrin, 4,4-dihydroxypyrazoline-5-dione and dihydroxyacetone. The preferred self-tanning agent used together with the penetration enhancer is dihydroxyacetone (DHA). The amount of self-tanning agents ranges from 0.05 to 10 percent by weight preferably 0.1 to 5 percent by weight based on the total weight of the cosmetic composition.

In general, whitening agents need to penetrate into the skin to unfold their function, many contain tyrosine inhibitors which prevent the formation of melanin and must therefore act upon the melanocytes of the skin. Examples of whitening agents are: arbutin, ferulic acid, kojic acid, coumaric acid, plant extracts and ascorbic acid (vitamin C).

### c) Anti-acne actives

Anti-acne actives such as anti-inflammatory agents, antibiotics, retinoids, anti-bacterial and keratolytic agents often cause undesirable side-effects. Therefore it is advantageous if the dose of these actives can be diminished due to an improved penetration and distribution in the upper skin layer, the sebocytes and/or pores of the skin.

The anti-acne actives used in combination with a penetration enhancer are chosen from the group consisting of alpha-hydroxy acids (AHAs) including glycolic and lactic acid; beta-hydroxy acid such as salicylic acid; benzoyl peroxide; hydroxyquinoline; sulfur; antibiotics such as for example tetracycline, dapzone, doxycycline, minocycline, erythromycin, clindamycin; azelaic acid; retinoids; isotretinoin; vitamin-A-derived topical retinoids, including tretinoin, isotretinoin, motretinide, adapalene and tazarotene; nicotinamide, allantoin and plant extracts.

### d) Anti-ageing actives

A number of keratolytic agents used as anti-acne actives are also known as anti-ageing actives. Especially alpha- and beta-hydroxy acids and retinoids and retinoic acid. Another class of anti-ageing actives encompasses vitamins, proteinhydrolysates, peptides and modified peptides, beta-glucans and plant extracts.

### e) Vitamins

Vitamins, provitamins or vitamin precursors of the vitamin B group or derivatives thereof and the derivatives of 2-furanone which are preferably to be employed according to the invention include, inter alia:
Vitamin B1, trivial name thiamine, chemical name 3-[(4'-amino-2'-methyl-5'-pyrimidinyl)methyl]-5-(2-hydroxyethyl)-4-methylthiazolium chloride,
Vitamin B2, trivial name riboflavin, chemical name 7,8-dimethyl-10-(1-D-ribityl)-benzo[g]pteridine-2,4(3H,10H)-dione. Riboflavin occurs e.g. in whey in the free form, and other riboflavin derivatives can be isolated from bacteria and yeasts. A stereoisomer of riboflavin which is likewise suitable according to the invention is the lyxoflavin which can be isolated from fish meal or liver and carries a D-arabityl radical instead of the D-ribityl radical. Vitamin B3. The compounds nicotinic acid and nicotinamide (niacinamide) are often mentioned by this name. Nicotinamide is preferred according to the invention.
Vitamin B5 (pantothenic acid and panthenol). Panthenol is preferably employed. Derivatives of panthenol which can be employed according to the invention are, in particular, the esters and ethers of panthenol and cationically derivatized panthenols. In a further preferred embodiment of the invention, derivatives of 2-furanone can also be employed in addition to pantothenic acid or panthenol. Particularly preferred derivatives are the substances, which are also commercially obtainable, dihydro-3-hydroxy-4,4-dimethyl-2(3H)-furanone with the trivial name pantolactone (Merck), 4-hydroxymethyl-butyrolactone (Merck), 3,3-dimethyl-2-hydroxy-butyrolactone (Aldrich) and 2,5-dihydro-5-methoxy-2-furanone (Merck), all stereoisomers expressly being included.

These compounds advantageously impart to the dermocosmetics according to the invention moisture-donating and skin soothing properties.

Vitamin B6, by which is understood not a uniform substance but the derivatives of 5-hydroxymethyl-2-methylpyridin-3-ol known by the trivial names pyridoxine, pyridoxamine and pyridoxol.

Vitamin B7 (biotin), also called vitamin H or "skin vitamin". Biotin is (3aS,4S,6aR)-2-oxohexahydro¬thienol[3,4-d]imidazole-4-valeric acid.

Panthenol, pantolactone, nicotinamide and biotin are particularly preferred vitamins according to the invention.

### f) Hair modulating agents

Hair modulating agents encompass
- hair growth stimulating agents which induce the growth of a new hair cycle, increase the growth rate, or increase the width of the hair shaft;
- agents with effects on the hair such as increasing hair volume, hair shine and hair strength and hair thickness;
- agents with effects on the hair follicle and
- hair growth inhibiting agents

In order to be effective hair modulating agents usually have to penetrate transdermally and transfollicularly as far as the hair bulb, so that the addition of penetration enhancers directly increases the effectiveness of hair modulating agents.

Most of the hair modulating agents are hair growth stimulating actives which prevent and slow down hair loss. Medical treatment with hair growth stimulating actives is often related to the use of drugs including but not limited to minoxidil, finasteride, dutasteride, bicalutamide, flutamide, progesterone derivatives or pregnane derivatives.

Other hair growth stimulators used in cosmetic compositions are zinc and zinc salts, unsaturated fatty acids, procyanidin oligomers, gamma-linolenic acid, biotin, placenta extract, pantothenic acid and derivatives thereof, prostaglandins such as bimatoprost, methylamido dihydro noralfaprostal and/or tafluprost; magnesium, allantoin, theophylline, and plant extracts including but not limited to extracts from barley, grape seed, apple seed, saw palmetto, uva-ursi, green tea, propolis, eleuthero root, pine bark.

Purin and purin derivatives are known to reduce hair loss.

Known scalp circulation promoters for example for the treatment of alopecia include but are not limited to acetylcholine derivatives, nicotinic acid derivatives, plant extracts from pepper, ginseng, swertia herb.

Suitable agents with effects on the hair follicle are taurin and taurin derivatives or L-carnitine or L-carnitine derivatives.

Hair growth inhibiting agents are mainly used for cosmetical reasons. They include but are not limited to niacinamid, thiazolidinon derivatives, proteinkinase C inhibitors , ornithine decarboxylase inhibitors, salts of trihydroxypurin; a combination of soy protein hydrolysate, urea, salicylic acid, willow extract, hamamelis extract, arnica extract, hypericum extract, salix alba extract and menthol marketed as Pilinhib® (BASF Beauty Care Solutions)

### g) Deodorizing or anti-perspirant agents

Cosmetic deodorants counteract, mask or remove body odours. Body odours arise as a result of the effect of skin bacteria on apocrine perspiration, with the formation of degradation products which have an unpleasant odour. Accordingly, deodorants comprise active ingredients which act as antimicrobial agents, odour absorbers and/or enzyme inhibitors. Suitable antimicrobial agents are, in principle, all substances effective against gram-positive bacteria, such as, for example, 4-hydroxybenzoic acid and its salts and esters, N-(4-chlorophenyl)-N'-(3,4-dichlorophenyl)urea, 2,4,4'-trichloro-2'-hydroxydiphenyl ether (triclosan), 4-chloro-3,5-dimethylphenol, 2,2'-methyl-enebis(6-bromo-4-chlorophenol), 3-methyl-4-(1-methylethyl)phenol, 2-benzyl-4-chlorophenol, 3-(4-chlorophenoxy)-1,2-propanediol, 3-iodo-2-proponyl butylcarbamate, chlorhexidine, 3,4,4'-trichlorocarbanilide (TTC), antibacterial fragrances, thymol, thyme oil, eugenol, oil of cloves, menthol, mint oil, farnesol, phenoxy-ethanol, glycerol monocaprate, glycerol monocaprylate, glycerol monolaurate (GML), diglycerol monocaprate (DMC), salicylic acid N-alkylamides, such as, for example, N-octylsalicylamide or N-decylsalicylamide.

Suitable enzyme inhibitors are, for example, esterase inhibitors. These are preferably trialkyl citrates, such as trimethyl citrate, tripropyl citrate, triisopropyl citrate, tributyl citrate and, in particular, triethyl citrate (Hydagen® CAT). The substances inhibit enzyme activity, thereby reducing the formation of odour. Other substances which are suitable esterase inhibitors are sterol sulphates or phosphates, such as, for example, lanosterol, cholesterol, campesterol, stigmasterol and sitosterol sulphate or phosphate, dicarboxylic acids and esters thereof, such as, for example, glutaric acid, monoethyl glutarate, diethyl glutarate, adipic acid, monoethyl adipate, diethyl adipate, malonic acid and diethyl malonate, hydroxycarboxylic acids and esters thereof, such as, for example, citric acid, malic acid, tartaric acid or diethyl tartrate, and zinc glycinate.

Suitable odour absorbers are substances which are able to absorb and largely retain odour-forming compounds. They lower the partial pressure of the individual components, thus also reducing their rate of diffusion. It is important that in this process perfumes must remain unimpaired. Odour absorbers are not effective against bacteria. They comprise, for example, as main constituent, a complex zinc salt of ricinoleic acid or specific, largely odour-neutral fragrances which are known to the person skilled in the art as "fixatives", such as, for example, extracts of labdanum or styrax or certain abietic acid derivatives. The odour masking agents are fragrances or perfume oils, which, in addition to their function as odour masking agents, give the deodorants their respective fragrance note. Perfume oils which may be mentioned are, for example, mixtures of natural and synthetic fragrances. Natural fragrances are extracts from flowers, stems and leaves, fruits, fruit peels, roots, woods, herbs and grasses, needles and branches, and resins and balsams. Also suitable are animal raw materials, such as, for example, civet and castoreum. Typical synthetic fragrance compounds are products of the ester, ether, aldehyde, ketone, alcohol and hydrocarbon type. Fragrance compounds of the ester type are, for example, benzyl acetate, p-tert-butylcyclohexyl acetate, linalyl acetate, phenylethyl acetate, linalyl benzoate, benzyl formate, allyl cyclohexylpropionate, styrallyl propionate and benzyl salicylate. The ethers include, for example, benzyl ethyl ether, and the aldehydes include, for example, the linear alkanals having 8 to 18 carbon atoms, citral, citronellal, citronellyloxy-acetaldehyde, cyclamen aldehyde, hydroxycitronellal, lilial and bourgeonal, the ketones include, for example, the ionones and methyl cedryl ketone, the alcohols include anethole, citronellol, eugenol, isoeugenol, geraniol, linalool, phenylethyl alcohol and terpineol, and the hydrocarbons include mainly the terpenes and balsams. Preference is, however, given to using mixtures of different fragrances which together produce a pleasing fragrance note. Essential oils of relatively low volatility, which are mostly used as aroma components, are also suitable as perfume oils, e.g. sage oil, camomile oil, oil of cloves, melissa oil, mint oil, cinnamon leaf oil, linden blossom oil, juniper berry oil, vetiver oil, olibanum oil, galbanum oil, labdanum oil and lavandin oil. Preference is given to using bergamot oil, dihydromyrcenol, lilial, lyral, citronellol, phenylethyl alcohol, hexylcinnamaldehyde, geraniol, benzylacetone, cyclamen aldehyde, linalool, boisambrene forte, ambroxan, indole, hedione, sandelice, lemon oil, mandarin oil, orange oil, allyl amyl glycolate, cyclovertal, lavandin oil, clary sage oil, damascone, geranium oil bourbon, cyclohexyl salicylate, iso-E-super, Fixolide NP, evernyl, iraldein gamma, phenylacetic acid, geranyl acetate, benzyl acetate, rose oxide, romilat, irotyl and floramat alone or in mixtures.

### Antiperspirants

Antiperspirants reduce the formation of perspiration by influencing the activity of the eccrine sweat glands, thus counteracting underarm wetness and body odor. Aqueous or anhydrous formulations of antiperspirants typically comprise astringent active ingredients.

Astringent antiperspirant active ingredients are primarily salts of aluminium, zirconium or of zinc. Such suitable antihydrotic active ingredients are, for example, aluminium chloride, aluminium chlorohydrate, aluminium dichlorohydrate, aluminium sesquichlorohydrate and complex compounds thereof, e.g. with 1,2-propylene glycol, aluminium hydroxyallantoinate, aluminium chloride tartrate, aluminium zirconium trichlorohydrate, aluminium zirconium tetrachlorohydrate, aluminium zirconium pentachlorohydrate and complex compounds thereof, e.g. with amino acids, such as glycine.

### Cosmetic and/or pharmaceutical compositions

The penetration enhancer according to the invention is used in cosmetic or other topical compositions, preferably compositions of emulsion type, most preferably capryloyl alanine ethylester is used as penetration enhancer in W/O type emulsions. These cosmetic or other topical compositions comprise the penetration enhancer in combination with at least one constituent which differs therefrom and is chosen from cosmetically active compounds, emulsifiers, surfactants, preservatives, perfume oils, thickeners, hair polymers, hair and skin conditioners, graft polymers, water-soluble or dispersible silicone-containing polymers, gelling agents, care agents, coloring agents, tinting agents, dyestuffs, pigments, agents for imparting consistency, humectants, re-oiling agents, collagen, protein hydrolysates, lipids, antioxidants, defoamers, antistatics, emollients and softeners.

Antioxidants are advantageously chosen from the group consisting of amino acids (e.g. glycine, histidine, tyrosine, tryptophan) and derivatives thereof, imidazoles (e.g. urocanic acid) and derivatives thereof, peptides, such as D,L-carnosine, D-carnosine, L-carnosine and derivatives thereof (e.g. anserine), carotenoids, carotenes (e.g. β-carotene, lycopene) and derivatives thereof, chlorogenic acid and derivatives thereof, liponic acid and derivatives thereof (e.g. dihydroliponic acid), aurothioglucose, propyl-thiouracil and other thiols (e.g. thioredoxin, glutathione, cysteine, cystine, cystamine and glycosyl, N-acetyl, methyl, ethyl, propyl, amyl, butyl and lauryl, palmitoyl, oleyl, γ-linoleyl, cholesteryl and glyceryl esters thereof) as well as salts thereof, dilauryl thiodipropionate, distearyl thiodipropionate, thiodipropionic acid and derivatives thereof (esters, ethers, peptides, lipids, nucleotides, nucleosides and salts) as well as sulfoximine compounds (e.g. buthionine sulfoximine, homocysteine sulfoximine, buthionine sulfones, penta-, hexa-, heptathionine sulfoximine) in very low tolerated dosages (e.g. pmol to µmol/kg), furthermore (metal) chelators, (e.g. α-hydroxy-fatty acids, palmitic acid, phytic acid, lactoferrin), α-hydroxy acids (e.g. citric acid, lactic acid, malic acid), humic acid, bile acid, bile extracts, bilirubin, biliverdin, EDTA and derivatives thereof, unsaturated fatty acids and derivatives thereof (e.g. γ-linolenic acid, linoleic acid, oleic acid), folic acid and derivatives thereof, ubiquinone and ubiquinol and derivatives thereof, vitamin C and derivatives thereof (e.g. sodium ascorbate, ascorbyl palmitate, Mg ascorbyl phosphate, ascorbyl acetate), tocopherol and derivatives (e.g. vitamin E acetate, tocotrienol), vitamin A and derivatives (vitamin A palmitate) as well as coniferylbenzoate of benzoin resin, rutic acid and derivatives thereof, α-glycosylrutin, ferulic acid, furfurylidene, carnosine, butylhydroxytoluene, butylhydroxyanisole, nordihydroguaiac resin acid, nordihydroguaiaretic acid, trihydroxybutyrophenone, uric acid and derivatives thereof, mannose and derivatives thereof, zinc and derivatives thereof (e.g. ZnO, ZnSO4), selenium and derivatives thereof (e.g. selenium methionine) and stilbenes and derivatives thereof (e.g. stilbene oxide, trans-stilbene oxide)

### Production process of capryloyl alanine ethylester

The production of capryloyl alanine ethyl ester (KE4610) is a two step-process:
Step 1: Acylation of alanine with capryloyl chloride according to the Schotten-Baumann method in aqueous solution.

120 kg (0.74 kmol) of capryloyl chloride is dosed to a intensively stirred aqueous solution of 66 kg (0.74 kmol) alanine in 100kg of water and 30kg of ethanol at 15°C over a time period of 4-5h. The pH-value is kept at 11-12 by constantly feeding of a 20% NaOH (approx.140 kg). The temperature should not exceed 25°C, the pH-value should not be lower than 10.5. After complete dosage of the capryloyl chloride the reaction mixture is post-reacted at 20-25°C and a pH of min. 9 for approx. 2h.

This mixture is heated up to 70°C under stirring for 1 h and then adjusted to a pH of 1.8-2.3 by adding approx. 80 kg of a 30 % solution of HCI in water. After stopping the stirrer the system starts to separate into a aqueous salt solution and an organic layer, containing 85-90 % capryloyl alanine, caprylic acid (hydrolysis product), ethanol and some water. The aqueous phase is removed and the organic layer (approx. 205 kg) is further processed in step 2.

### Step 2: Esterification and purification

The organic layer is subjected to a esterification by removing water/ethanol by distillation, adding fresh ethanol until the desired degree of esterification is reached (at least 80-90 %), which can be controlled by GC-analysis or by determining the remaining acid value. The esterification mixture is then subjected to a fractionated distillation. The low boilers water, ethanol and caprylic acid are removed at temperatures up to 105°C at < 1 mbar, the final product can be distilled at 137-140°C at < 1 mbar. In order to obtain a high purity of the ester, the mixture can be adjusted prior to the distillation to pH = 7 by adding a aliquot amount of soda or NaOH in order to transfer volatile acids to the not distillable salts. The preferred product has an acid value of < 1 after distillation.

The efficacy of the penetration enhancer is independent of the production process so that other production processes for capryloyl alanine ethyl ester known by the skilled person are possible and suitable.

### 4. Examples

### 4.1 Penetration Study with the enhancer applied directly to the skin (preincubation)

The aim of the present study was to determine the potency of three test substances (one being the closest prior art penetration enhancer) to act as skin permeation enhancer in cosmetic formulations:
- Dimethyl isosorbide (DMI) a well-known penetration enhancer - used in cosmetics
- Penetration Enhancer 1 (PE1) - capryloyl alanine ethylester
- Penetration Enhancer 2 (PE2) - closest prior art molecule dodecyloyl alanine ethylester

The tests were performed by an independent test institute specialized in penetration testing. Formulation 1 (W/O formulation) is the formulation used and does not contain a penetration enhancer. The skin was pre-incubated with the selected penetration enhancers (DMI, PE1 and PE2) and after incubation time of 30 min formulation was removed from the skin surface and formulation 1 spiked with radiolabeled ³H-caffeine or ³H-Vitamin E acetate was applied.

**Tab. 1: Experimental setup:**

| **No.** | **Penetration Enhancer** | **Caffeine** | **Vit. E** | **Description** |
|---|---|---|---|---|
| A | None | 1 | 0 | Control |
| B | DMI | 1 | 0 | Test formulation |
| C | PE1 (C8) | 1 | 0 | Test formulation |
| D | PE2 (C12) | 1 | 0 | Test formulation |
| E | None | 0 | 1 | Control |
| F | DMI | 0 | 1 | Test formulation |
| G | PE1 (C8) | 0 | 1 | Test formulation |
| H | PE2 (C12) | 0 | 1 | Test formulation |

The impact of the three penetration enhancers on the skin absorption rate of caffeine and Vitamin E acetate (representative hydrophilic and lipophilic cosmetic actives, respectively) incorporated into a w/o formulation was investigated. Radiolabeled caffeine and Vitamin E acetate were used as markers. The in vitro skin permeation studies were performed with dermatomized human skin (approximately 500 µm in thickness) over a period of 24 h in triplicate. Samples from the acceptor compartment were taken at four sampling times (1, 3, 6 and 24 h) to screen the permeation enhancement effect.

The transdermal absorption of the test compounds ³H-caffeine and ³H-Vitamin E acetate was monitored by measuring the amount of radio labeled substances in the acceptor medium.

The test compounds were quantified by liquid scintillation counting. Before starting the in vitro permeation experiments, the linearity of scintillation counting for both isotopes was proven.

### Description of the test method

### Materials and methods

**Tab. 2: Formulation 1 was used as a W/O-formulation (formulation 1):**

| Raw Materials | | Weight (g) |
|---|---|---|
| 1) | Emulgade 165 | 14,00 |
| | Glyceryl stearate (and) PEG-100 Stearate (BASF) | |
| | Cetiol CC | 20,00 |
| | Di-n-Octylcarbonate (BASF) | |
| | Cetiol Sensoft | 24,00 |
| | Propylheptyl Caprylate (BASF) | |
| | Cetiol B | 24,00 |
| | Dibutyl Adipate (BASF) | |
| | Phenonip XB | 2,40 |
| | Preservative | |
| | | |
| 2) | Water demin. | 296,80 |
| | Glycerine | 12,00 |
| | Rheocare C+ | 0,40 |
| | Carbomer (BASF) | |
| | Caffeine | 4,00 |
| | | |
| 3) | NaOH sol 25% | 0,40 |
| | | |
| 4) | Copherol 1250C | 2,00 |
| | Tocopheryl acetate (BASF) | |

In accordance to the SCCP Guidelines of the European Commission the excised human skin from surgery is an appropriate in vitro test system to simulate the permeation and penetration processes of a compound into and across human skin under in vivo conditions.

For the penetration study, the human skin (female abdomen skin) of one donor which was dermatomized to a thickness of 500 ± 100 µm was used and tests were conducted in triplicate. Full-thickness skin was prepared from the skin biopsies and stored at -20°C. According to the OECD Guideline, storage of skin at -20°C for a period of up to 466 days does not alter its permeability. The skin was used immediately after thawing. Repeated freeze-thaw cycles were avoided.

Formulation 1 (without penetration enhancer) was spiked with radiolabeled ³H-caffeine (specific activity: 60 Ci/mmol) and ³H-Vitamin E acetate (specific activity: 50 Ci/mmol) and applied as control directly to the skin. The radio labeled samples were prepared as follows: Formulation 1 was spiked with either radiolabeled ³H-caffeine or ³H-Vitamin E acetate resulting in 2960000 cpm/50µl or 1264557 cpm/50µl, respectively. One of the three different penetration enhancers (DMI, PE1 and PE2) was applied to the skin prior to the application of the radioactively spiked W/O-formulation.

Krebs Ringer buffer (KRB, pH=7.4) was prepared according to an internal standard operation procedure. KRB was used as the acceptor medium for ³H-caffeine. For ³H-Vitamin E acetate, KRB supplemented with 2 % bovine serum albumin (BSA) was used as the acceptor medium. Solutions of ³H-caffeine and ³H-Vitamin E acetate were used to validate the detection method and to assess linearity.

### Transport Studies

Permeation study was performed in four fold determination with the test and the reference item at human skin. For the transport studies the dermatomized skin was cut with a punch of 24 mm diameter and the thickness of the skin was measured at five points for each sample. Subsequently the skin pieces were fastened on cylindrical glass Franz cells which are employed as diffusion chambers. The lower (acceptor) chamber has a volume of approximately 12 ml, while the volume of the upper (donor) chamber is variable. The skin was inserted with the dermal layer facing downwards so that the skin's corneous layer is uppermost..

Prior to application of the formulation the penetration enhancers were applied to and spread on the skin (20 µl each). This method was used in order to avoid effects which occur due to possible interactions of the penetration enhancer with components in the cosmetic composition.

PE2 (C12) which is solid at room temperature, was gently melted in a water bath (at 35 to 40°C) and applied as liquid. After 30 min of incubation the liquid penetration enhancers were gently removed with cotton balls. In the case of PE2 which solidified on the skin, the solid film was removed with a pincer. Thereafter 50 µl of the formulations were applied at the surface of the skin. The acceptor medium was thermostated at 32 °C and continuously stirred at a rate of 400 rpm. The diffusion area of skin was approximately 1.8 cm². Permeation through the skin into the acceptor medium was monitored over a period of 24 hours. The acceptor medium was sampled at 4 different time points, after 1, 3, 6 and 24 hours, by withdrawing samples of 200 µl. After withdrawing a sample volume, the acceptor medium was refilled with pre-warmed KRB/KRB + 2 % BSA. The concentration of active ingredients in the acceptor chamber was corrected to take the dilution introduced by sampling into account. Taken samples were mixed with 210 µl or 500 µl of scintillation cocktail for at least 1 hour at 200 rpm and radioactivity measured by scintillation counting.

Results were presented as active release into accepter medium [cpm cm⁻²] plotted against the time. By linear regression the amount of active ingredient in unknown samples was calculated using the corresponding calibration.

### Results

The results of the permeability studies are summarized in the following tables.

**Tab. 3:Values of the average cumulative transport, permeation coefficient and enhancement ratio for ³H-caffeine after 24 hours with standard deviations (n=3).**

| | Reference (w/o) | DMI | PE1 | PE2 |
|---|---|---|---|---|
| Average cumulative transport [dpm/cm²] | 84675 | 203120 | 414024 | 262929 |
| S | 15403 | 631 | 72578 | 42151 |
| RSD [%] | 18.19 | 31 | 17.53 | 16.03 |
| Enhancement Ratio | - | 2.40 | 4.89 | 3.11 |

The highest permeation enhancing effect was detected with PE1. The transport from this formulation is approximately 5 times higher compared to the control without any penetration enhancer and substantially higher than DMI or PE2.

**Tab. 4:Values of the average cumulative transport, permeation coefficient and enhancement ratio for ³H-Vitamin E acetate at 24 hours of permeability study with standard deviations (n=3).**

| | Reference (w/o) | DMI | PE1 | PE2 |
|---|---|---|---|---|
| Average cumulative transport [dpm/cm²] | 2560 | 2578 | 2712 | 1814 |
| S | 123 | 172 | 427 | 75 |
| RSD [%] | 4.82 | 6.67 | 15.75 | 4.11 |
| Enhancement Ratio | - | 1.01 | 1.06 | 0.70 |

For Vitamin E acetate a much slower transport was detectable. The reason might be the insufficient release of this lipophilic substance from the formulation and/or the binding to lipophilic structures within the skin. The applied penetration enhancers DMI and PE1 showed only a slight penetration enhancing effect during the 24 h detection time. However, PE 2 reduced the transport of Vitamin E acetate and actually acts as a retarding agent. In comparison to the prior state of the art molecule PE2 (dodecyl alanine ethylester, PE1 (capryloyl alanine ethylester) enhanced the penetration effects by 1,5fold.

### Conclusion

The most effective permeation enhancer is PE1 for the hydrophilic substance caffeine incorporated in the w/o formulation. DMI enhanced the caffeine transport around twice and PE2 enhanced the transport of caffeine by approximately 3 times.

The transport of the lipophilic Vitamin E acetate incorporated in the same w/o formulation is not significantly enhanced by the used permeation enhancers. Nevertheless, the use of capryloyl alanine ethylester as a penetration enhancer is still favorable compared with DMI or dodecyloyl alanine ethylester as it does not show a retarding effect on the penetration of the lipophilic molecule Vitamin E acetate.

### 4.2 Penetration Study with Enhancer incorporated into W/O formulation (incorporated)

The described study was partly repeated in a similar experimental setup with three test substances to act as skin permeation enhancer when incorporated into the cosmetic formulation and not applied directly to the skin:
- Dimethyl isosorbide (DMI) a well-known penetration enhancer - used in cosmetics
- Penetration Enhancer 1 (PE1) - capryloyl alanine ethylester
- Penetration Enhancer 2 (DGME) -prior art penetration enhancer Transcutol (Gattefossee), diethylene glycol mono ethyl ether

Formulation 1 (W/O formulation) served as a basic formulation and as a reference without any penetration enhancer. This time the skin was not pre-incubated with the selected penetration enhancers (DMI, PE1 and DGME), but 5 % of the respective penetration enhancer was incorporated into formulation 1 and the resulting formulations were spiked once again with radiolabeled ³H-caffeine or ³H-Vitamin E acetate.

**Tab. 5: Composition of W/O-formulations including penetration enhancer:**

| Raw Materials | | Weight (g) |
|---|---|---|
| 1) | Emulgade 165 | 14,00 |
| | Glyceryl stearate (and) PEG-100 Stearate (BASF) | |
| | Cetiol CC | 20,00 |
| | Di-n-Octylcarbonate (BASF) | |
| | Cetiol Sensoft | 24,00 |
| | Propylheptyl Caprylate (BASF) | |
| | Cetiol B | 24,00 |
| | Dibutyl Adipate (BASF) | |
| | Penetration enhancer (DMI or PE1 or DGME) | 20,00 |
| | Phenonip XB | 2,40 |
| | Preservative | |
| | | |
| 2) | Water demin. | 276,80 |
| | Glycerine | 12,00 |
| | Rheocare C+ | 0,40 |
| | Carbomer (BASF) | |
| | Caffeine | 4,00 |
| | | |
| 3) | NaOH sol 25% | 0,40 |
| | | |
| 4) | Copherol 1250C | 2,00 |
| | Tocopheryl acetate (BASF) | |

### Results:

The results of the permeability study are summarized in table 6.

**Tab. 6: Values of the average cumulative transport, permeation coefficient and enhancement ratio for ³H-caffeine at 24 hours of permeability study with standard deviations (n=4). Permeation from w/o formulation without and with penetration enhancer.**

| n=4 | Reference formulation 1 without enhancer | with 5 % DGME | with 5 % DMI | with 5 % PE1 |
|---|---|---|---|---|
| Average cumulative transport [cpm/cm2] | 247.789 | 278.404 | 212.435 | 342.593 |
| SD | 53.207 | 43.897 | 32.298 | 71.196 |
| RSD [%] | 21 | 16 | 15 | 21 |
| Enhancement Ratio | 1 | 1.12 | 0.86 | 1.38 |

### Conclusion:

Also if the penetration enhancer is incorporated into the W/O formulation the most effective permeation enhancer for the hydrophilic marker compound caffeine is PE1. DMI lead to a slightly decreased transport of caffeine, and DGME slightly enhanced the transport compared to the reference formulation without any enhancer.

## Claims

1. Cosmetic composition comprising capryloyl alanine ethylester and at least one active agent.

2. Cosmetic composition according to claim 1 **characterised in that** it contains 0.1 to 90 weight % of capryloyl alanine ethylester based on the total composition.

3. Cosmetic composition according to claim 1 or claim 2, **characterized in that** it is an emulsion composition.

4. Cosmetic composition according to claim 1, **characterized in that** the active agent is selected from the group consisting of UV-filters, anti-acne actives, anti-aging actives, anti-inflammatory agents, hair growth modulating agents, agents with effects on the hair or hair follicle, antiperspirant or deodorizing agents, whitening agents, self-tanning agents, peptides, oligopeptides, proteins, acetylsalicylic acid, atropine, azulene, hydrocortisone and derivatives thereof and vitamins.

5. Cosmetic composition comprising capryloyl alanine ethylester and at least one UV-filter.

6. Cosmetic composition comprising capryloyl alanine ethylester and at least one self-tanning agent or at least one whitening agent.

7. Cosmetic composition comprising capryloyl alanine ethylester and at least one antiperspirant and/or deodorizing agent.

8. Cosmetic composition comprising capryloyl alanine ethylester and at least one anti-acne active and/or at least one anti-inflammatory active.

9. Cosmetic composition comprising capryloyl alanine ethylester and at least one anti-ageing active.

10. Cosmetic composition comprising capryloyl alanine ethylester and vitamins.

11. Cosmetic composition comprising capryloyl alanine ethylester and at least one hair growth modulating active.

12. Non-therapeutic use of capryloyl alanine ethylester as a penetration enhancer for cosmetically active substances.

13. Non-therapeutic method of enhancing the skin penetration of cosmetically active substances by topically applying a composition comprising capryloyl alanine ethylester.

## Patentansprüche

1. Kosmetische Zusammensetzung, umfassend Capryloylalaninethylester und mindestens einen Wirkstoff.

2. Kosmetische Zusammensetzung nach Anspruch 1, **dadurch gekennzeichnet, dass** sie 0,1 bis 90 Gew.-% Capryloylalaninethylester, bezogen auf die gesamte Zusammensetzung, enthält.

3. Kosmetische Zusammensetzung nach Anspruch 1 oder 2, **dadurch gekennzeichnet, dass** es sich um eine Emulsionszusammensetzung handelt.

4. Kosmetische Zusammensetzung nach Anspruch 1, **dadurch gekennzeichnet, dass** der Wirkstoff aus der Gruppe bestehend aus UV-Filtern, Mitteln gegen Akne, Mitteln gegen das Altern, entzündungshemmenden Mitteln, Mitteln zur Modulierung des Haarwuchses, Mitteln mit Wirkungen auf das Haar oder Haarfolikel, Antitranspirantien oder Desodorantien, Aufhellern, Selbstbräunungsmitteln, Peptiden, Oligopeptiden, Proteinen, Acetylsalicylsäure, Atropin, Azulen, Hydrocortison und Derivaten davon und Vitaminen ausgewählt ist.

5. Kosmetische Zusammensetzung, umfassend Capryloylalaninethylester und mindestens einen UV-Filter.

6. Kosmetische Zusammensetzung, umfassend Capryloylalaninethylester und mindestens ein Selbstbräunungsmittel oder mindestens einen Aufheller.

7. Kosmetische Zusammensetzung, umfassend Capryloylalaninethylester und mindestens ein Antiperspirant und/oder Desodorant.

8. Kosmetische Zusammensetzung, umfassend Capryloylalaninethylester und mindestens ein Mittel gegen Akne und/oder mindestens ein entzündungshemmendes Mittel.

9. Kosmetische Zusammensetzung, umfassend Capryloylalaninethylester und mindestens ein Mittel gegen das Altern.

10. Kosmetische Zusammensetzung, umfassend Capryloylalaninethylester und Vitamine.

11. Kosmetische Zusammensetzung, umfassend Capryloylalaninethylester und mindestens ein Mittel zur Modulierung des Haarwuchses.

12. Nichttherapeutische Verwendung von Capryloylalaninethylester als Penetrationförderer für kosmetische Wirkstoffe.

13. Nichttherapeutisches Verfahren zur Verbesserung der Hautpenetration von kosmetischen Wirkstoffen durch topisches Aufbringen einer Zusammensetzung, die Capryloylalaninethylester umfasst.

## Revendications

1. Composition cosmétique comprenant du capryloyl alanine éthylester et au moins un agent actif.

2. Composition cosmétique selon la revendication 1, **caractérisée en ce qu'**elle contient de 0,1 à 90% en poids de capryloyl alanine éthylester, sur la base de la composition totale.

3. Composition cosmétique selon la revendication 1 ou la revendication 2, **caractérisée en ce qu'**il s'agit d'une composition en émulsion.

4. Composition cosmétique selon la revendication 1, **caractérisée en ce que** l'agent actif est choisi dans le groupe constitué par les filtres anti-UV, les principes actifs anti-acné, les principes actifs anti-vieillissement, les agent anti-inflammatoires, les agents de modulation de la croissance capillaire, les agents ayant des effets sur les poils ou les follicules capillaires, les agents antitranspirants ou désodorisants, les agents de blanchiment, les agents autobronzants, les peptides, les oligopeptides, les protéines, l'acide acétylsalicylique, l'atropine, l'azulène, l'hydrocortisone et des dérivés de ceux-ci, et les vitamines.

5. Composition cosmétique, comprenant du capryloyl alanine éthylester et au moins un filtre anti-UV.

6. Composition cosmétique, comprenant du capryloyl alanine éthylester et au moins un agent autobronzant ou au moins un agent de blanchiment.

7. Composition cosmétique, comprenant du capryloyl alanine éthylester et au moins un agent antitranspirant et/ou désodorisant.

8. Composition cosmétique, comprenant du capryloyl alanine éthylester et au moins un principe actif anti-acné et/ou au moins un principe actif anti-inflammatoire.

9. Composition cosmétique, comprenant du capryloyl alanine éthylester et au moins un principe actif anti-vieillissement.

10. Composition cosmétique, comprenant du capryloyl alanine éthylester et des vitamines.

11. Composition cosmétique, comprenant du capryloyl alanine éthylester et au moins un principe actif de modulation de la croissance capillaire.

12. Utilisation non thérapeutique de capryloyl alanine éthylester comme améliorateur de pénétration pour des substances actives sur le plan cosmétique.

13. Méthode non thérapeutique destinée à améliorer la pénétration dans la peau de substances actives sur le plan cosmétique, par l'application par voie topique d'une composition comprenant du capryloyl alanine éthylester.
